Europäisches Patentamt

European Patent Office

Office européen des brevets

(11) Publication number: **0 437 622 A1**

# EUROPEAN PATENT APPLICATION
## published in accordance with Art. 158(3) EPC

(21) Application number: **90910190.9**

(22) Date of filing: **06.07.90**

(86) International application number:
**PCT/JP90/00873**

(87) International publication number:
**WO 91/00739 (24.01.91 91/03)**

(51) Int. Cl.⁵: **A61K 37/24**

(30) Priority: **07.07.89 JP 176436/89**

(43) Date of publication of application:
**24.07.91 Bulletin 91/30**

(84) Designated Contracting States:
**AT BE CH DE DK ES FR GB IT LI LU NL SE**

(71) Applicant: **KYOWA HAKKO KOGYO CO., LTD.**
**Ohtemachi Bldg., 6-1 Ohtemachi I-chome**
**Chiyoda-ku Tokyo 100(JP)**

(72) Inventor: **" NO, Yasuhiko 410-1, Nameri**
**Nagaizumi-cho Sunto-gun**
**Shizuoka 411(JP)**
Inventor: **TERAJIMA, Mitsuru 110-1, Honjuku**
**Nagaizumi-cho Sunto-gun**
**Shizuoka 411(JP)**
Inventor: **HAYAKAWA, Eiji**
**495-15, Chabatake Susono-shi**
**Shizuoka 410-11(JP)**

(74) Representative: **Vossius & Partner**
**Siebertstrasse 4 P.O. Box 86 07 67**
**W-8000 München 86(DE)**

(54) **STABLE MOTILIN PREPARATION.**

(57) A stable motilin preparation which comprises motilin and at least one pharmaceutically acceptable stabilizer selected from the group consisting of sugars, amino acids, inorganic salts, and proteins.

## TECHNICAL FIELD

The present invention relates to a motilin preparation containing a motilin and a stabilizer and which has excellent stability.

Motilin is a gastrointestinal hormone which stimulates gastrointestinal motility in hunger and is expected to be useful as a drug for, e.g., improving gastrointestinal motility.

## Background Art

In recent years, with the development of synthetic technology and recombinant DNA technology, it has become possible to produce many physiologically active proteins and peptides. Motilins in the present invention can be produced by peptide synthesis and recombinant DNA technology. No method is known for stabilizing motilin preparations.

Motilins are susceptible to influences of external factors and have poor stability, like general physiologically active peptides. For example, owing to temperature, humidity, oxygen and UV rays, motilins undergo physical or chemical changes such as inactivation, denaturation, aggregation, adsorption and oxidation to cause reduction in activity. Further, motilins are decomposed by protease produced by microorganisms.

## Disclosure of the Invention

The present invention is based on the finding that a motilin preparation can be improved in stability by adding a sugar, an amino acid, an inorganic salt and a protein and can be provided for practical use.

The present invention relates to a preparation comprising a motilin and at least one stabilizer selected from the group consisting of a sugar, an amino acid, an inorganic salt and a protein which are pharmaceutically acceptable and to a freeze-dried motilin-containing preparation obtained by freeze-drying a solution comprising a motilin and at least one stabilizer selected from the group consisting of a sugar, an amino acid, an inorganic salt and a protein which are pharmaceutically acceptable.

The motilins to be used in the present invention are not particularly limited, and usually usable ones such as swine motilin, canine motilin and human motilin [it has been confirmed that human motilin has the same structure as that of swine motilin, FEBS LETTER, 223, 1, 74 (1987)] can be used. In addition, there may also be used motilin derivatives, e.g., [13]leucine-swine motilin wherein the methionine at the 13-position of swine motilin is substituted with leucine (Japanese Published Unexamined Patent Application No. 71195/88). These motilins may be either in a free state or in the form of an organic or inorganic acid salt.

As the sugars which are used as the stabilizer for obtaining the stable motilin-containing preparation of the present invention, any of monosaccharides, oligosaccharides, polysaccharides, and phosphoric acid esters thereof may be utilized, and there is no particular restriction so long as they are pharmaceutically acceptable. Specific examples include sugar alcohols such as glycerine, mannitol, xylitol and sorbitol, acidic sugars such as glucuronic acid, iduronic acid, galacturonic acid, gluconic acid, mannuronic acid and ketoglycolic acid, hyaluronic acid and salts thereof, chondroitin sulfuric acid and salts thereof, heparin, inulin, chitin and derivatives thereof, chitosan and derivatives thereof, dextran, dextrin having an average molecular weight of 3,000 to 150,000, hydroxypropyl cellulose, hydroxypropyl starch, hydroxymethyl cellulose, sodium carboxymethyl cellulose and hydroxyethyl cellulose.

It is preferred that these sugars be used generally in the range of 0.01 to 10,000 parts by weight based on 1 part by weight of the motilin.

Examples of the amino acids which are used as the stabilizer for obtaining the stable motilin-containing preparation of the present invention include alanine, valine, leucine, isoleucine, proline, phenylalanine, tryptophan, serine, threonine, cysteine and salts thereof, asparagine, glutamine, aspartic acid and salts thereof, glutamic acid and salts thereof, lysine and salts thereof, and arginine and salts thereof.

It is preferred that these amino acids be used generally in the range of 0.01 to 10,000 parts by weight based on 1 part by weight of the motilin.

Examples of the inorganic acids which are used as the stabilizer for obtaining the stable motiline-containing preparation of the present invention include NaCl, KCl, LiCl, $MgCl_2$, $CaCl_2$, $SrCl_2$, NaBr, KBr and LiBr.

It is preferred that these inorganic acids be used generally in the range of 0.01 to 10,000 parts by weight based on 1 part by weight of the motilin.

Examples of the proteins which are used as the stabilizer for obtaining the stable motilin-containing preparation of the present invention include human serum albumin, human serum globulin, collagen, gelatin, acid-treated gelatin (average molecular weight: 7,000 to 100,000), and alkali-treated gelatin (average molecular weight: 7,000 to 100,000).

It is preferred that these proteins be used generally in the range of 0.01 to 10,000 parts by weight based on 1 part by weight of the motilin.

The pH controllers to be used in the present invention are not particularly limited but are those capable of maintaining the pH 3.0 to 9.0 and which are pharmaceutically acceptable. A preferred pH range for the motilin preparation in the present invention is 4.0 to 7.0, more preferably 4.0 to 5.5.

Specific examples of the pH controllers include acetic acid-sodium acetate, potassium hydrogenphthalate-sodium hydroxide, sodium secondary citrate-hydrochloric acid, glycine-sodium chloride-hydrochloric acid, sodium secondary citrate-sodium hydroxide, monopotassium phosphate-disodium phosphate, monosodium phosphate-disodium phosphate, monosodium phosphate-dipotassium phosphate, monopotassium phosphate-dipotassium phosphate, tartaric acid-sodium tartrate, lactic acid-sodium lactate, sodium Veronal-sodium acetate-hydrochloric acid, succinic acid-borax, potassium primary citrate-sodium hydroxide, potassium primary citrate-borax, disodium phosphate-citric acid, sodium acetate-hydrochloric acid, glutamic acid-sodium hydroxide, and aspartic acid-sodium hydroxide.

The motilin-containing preparation of the present invention may also contain, depending upon the purpose of the preparation, a preservative, a stabilizer, an antioxidant, an excipient, a binder, a disintegrator, a wetting agent, a lubricant, a coloring agent, an aromatic, a corrigent, a coating, a suspending agent, an emulsifier, a dissolution aid, a buffer, an isotonic agent, a plasticizer, a surfactant, an analgesic, etc. which are pharmaceutically acceptable.

Examples of the antioxidants include erythorbic acid, dibutylhydroxytoluene, butylhydroxyanisole, $\alpha$-tocopherol, L-ascorbic acid and salts thereof, L-ascorbic acid stearate, sodium hydrogensulfite, sodium sulfite, propyl gallate, triamyl gallate, sodium pyrophosphate, sodium metaphosphate, and chelating agents such as disodium ethylenediaminetetraacetate (EDTA).

The freeze-dried preparation of motilin can be obtained by conventional methods. That is, a motilin (0.1 to 10 mg/ml) is dissolved in an aqueous solution adjusted to pH 4.0 to 5.5 and the solution is filled into a suitable container, followed by freeze-drying by using a freeze-drier. For example, an aqueous solution of motilin is filled into a sterile glass vial, frozen at a temperature below -50°C for 4 hours, and then dried at -10°C for 36 hours in vacuum at 0.05 mbar. Then, the vial is subjected to secondary drying at 40°C for 8 hours. After completion of the drying, the vial is filled with sterile dry nitrogen gas until the atmospheric pressure is reached. Thereafter, the vial is stoppered with a rubber stopper for freeze-drying and then sealed with an aluminum cap. In this case, an excipient such as mannitol or lactose may be added to improve the appearance of a cake of the freeze-dried preparation.

The stabilized motilin-containing preparation of the present invention can be used for various modes of administration such as oral administration and parenteral administration by various injections, and various preparations can be realized depending upon the mode for administration. Examples of the preparations for administration are those for oral administration such as tablets, pills, capsules, granules and suspensions; solutions and suspensions for intravenous, intramuscular, subcutaneous or intracutaneous injections; freeze-dried preparations, and the like which are dissolved just before use to prepare injections; and preparations for transmucosal administration such as suppositories, nasal drops, and vaginal suppositories.

Example 1

In water were dissolved 10 mg of [13]leucine-swine motilin and a stabilizer shown in Table 1 to aseptically prepare 100 μg/ml aqueous solution of [13]leucine-swine motilin. The aqueous solution was aseptically filled and sealed in a glass vial to give a preparation of [13]leucine-swine motilin solution. The obtained preparations were stored in a thermostat at 60°C and the change with the passage of time was determined by the HPLC method. The results are shown in Table 1.

Table 1

|  |  | Stored at 60°C, pH 4.5 | |
|  | Concentration | Residual Activity (%) | |
| Stabilizer | mg/ml | 2 weeks | 1 month |
| None | - | 85.3 | 63.6 |
| Mannitol | 40 | 98.5 | 70.4 |
| Xylitol | 40 | 97.0 | 69.3 |
| Proline | 40 | 97.8 | 77.6 |
| Arginine | 40 | 93.4 | 70.1 |
| Alanine | 40 | 91.7 | 67.1 |
| NaCl | 8 | 88.0 | 67.1 |
| Gelatin | 1 | 92.9 | 71.7 |

Example 2

In distilled water for injection were dissolved 150 μg of [13]leucine-swine motilin and a stabilizer shown in Table 2 to aseptically prepare 2.5 ml of an aqueous solution. The aqueous solution was filled into a glass vial and freeze-dried to give a freeze-dried preparation of [13]leucine-swine motilin.

The obtained freeze-dried preparations of [13]leucine-swine motilin were stored in a thermostat at 80°C and the change with the passage of time was determined by the HPLC method. The results are shown in Table 2.

## Table 2

| Stabilizer | Amount mg | Stored at 80°C, pH 7.0 Residual Activity (%) 80°C, 7 days |
|---|---|---|
| None | - | 32.9 |
| Mannitol | 120 | 43.8 |
| Dextran T70 | 120 | 81.8 |
| Alanine | 120 | 58.1 |
| Arginine | 120 | 98.2 |
| Cysteine | 120 | 52.6 |
| Na glutamate | 120 | 56.1 |
| Lysine | 120 | 66.4 |
| Proline | 120 | 54.9 |
| Serine | 120 | 48.9 |
| NaCl | 24 | 57.9 |
| Gelatin | 3 | 77.3 |

Example 3

To 10 mg of [13]methionine-swine motilin was added the same stabilizer as used in Example 1 to aseptically prepare 100 $\mu$g/ml aqueous solution of [13]methionine-swine motilin. The aqueous solution was aseptically filled and sealed in a glass vial to give a preparation of [13]methionine-swine motilin solution. The obtained preparations were stored in a thermostat at 60°C and the change with the passage of time was determined by the HPLC method. The results are shown in Table 3.

## Table 3

| Stabilizer | Concentration mg/ml | Stored at 60°C, pH 4.5 Residual Activity (%) 2 weeks | 1 month |
|---|---|---|---|
| None | - | 86.0 | 64.6 |
| Mannitol | 40 | 97.3 | 72.7 |
| Xylitol | 40 | 98.1 | 70.4 |
| Proline | 40 | 98.5 | 78.1 |
| Arginine | 40 | 96.4 | 73.9 |
| Alanine | 40 | 82.2 | 69.3 |
| NaCl | 8 | 87.5 | 69.4 |
| Gelatin | 1 | 90.9 | 65.9 |

Example 4

In distilled water for injection were dissolved 150 µg of [13]methionine-swine motilin and the same stabilizer as used in Example 2 to aseptically prepare 2.5 ml of an aqueous solution. The aqueous solution was filled into a glass vial and freeze-dried to give a freeze-dried preparation of [13]methionine-swine motilin.

The obtained freeze-dried preparations of [13]methionine-swine motilin were stored in a thermostat at 80°C and the change with the passage of time was determined by the HPLC method. The results are shown in Table 4.

Table 4

| Stabilizer | Amount mg | Stored at 80°C, pH 7.0 Residual Activity (%) 80°C, 7 days |
|---|---|---|
| None | - | 28.9 |
| Mannitol | 120 | 40.5 |
| Dextran T70 | 120 | 83.7 |
| Alanine | 120 | 58.8 |
| Arginine | 120 | 99.0 |
| Cysteine | 120 | 67.3 |
| Na glutamate | 120 | 54.8 |
| Lysine | 120 | 69.1 |
| Proline | 120 | 52.9 |
| Serine | 120 | 45.6 |
| NaCl | 24 | 60.2 |
| Gelatin | 3 | 81.5 |

Claims

1. A motilin-containing preparation comprising a motilin and at least one stabilizer selected from a sugar, an amino acid, an inorganic salt and a protein which are pharmaceutically acceptable.

2. A freeze-dried motilin-containing preparation obtained by freeze-drying an aqueous solution comprising a motilin and at least one stabilizer selected from a sugar, an amino acid, an inorganic salt and a protein which are pharmaceutically acceptable.

3. A motilin-containing preparation or a freeze-dried motilin-containing preparation according to claim 1 or 2, wherein said stabilizer is contained in the range of 0.01 to 10,000 parts by weight based on 1 part by weight of the motilin.

4. A motilin-containing preparation or a freeze-dried motilin-containing preparation according to claim 1 or 2, wherein said sugar is selected from the group consisting of sugar alcohols such as glycerine, mannitol, xylitol, sorbitol and meglumine, and polysaccharides such as hyaluronic acid and salts

6

thereof, heparin, inulin, chitin and derivatives thereof, chitosan and derivatives thereof, dextran, dextrin having an average molecular weight of 3,000 to 150,000, hydroxypropyl cellulose, hydroxypropyl starch, hydroxymethyl cellulose, sodium carboxymethyl cellulose, and hydroxyethyl cellulose.

5. A motilin-containing preparation or freeze-dried motilin-containing preparation according to claim 1 or 2, wherein said amino acid is selected from the group consisting of alanine, valine, leucine, isoleucine, proline, phenylalanine, tryptophan, serine, threonine, cysteine, asparagine, glutamine, aspartic acid, glutamic acid, lysine, arginine and salts thereof.

6. A motilin-containing preparation or a freeze-dried motilin-containing preparation according to claim 1 or 2, wherein said protein is selected from the group consisting of human serum albumin, human serum globulin, collagen, gelatin, acid-treated gelatin and alkali-treated gelatin having an average molecular weight of 7,000 to 100,000.

7. A motilin-containing preparation or a freeze-dried motilin-containing preparation according to claim 1 or 2, which further contains a pH controller as a stabilizer.

# INTERNATIONAL SEARCH REPORT

International Application No PCT/JP90/00873

**I. CLASSIFICATION OF SUBJECT MATTER** (if several classification symbols apply, indicate all) *

According to International Patent Classification (IPC) or to both National Classification and IPC

Int. Cl$^5$    A61K37/24

**II. FIELDS SEARCHED**

| Minimum Documentation Searched [7] | |
|---|---|
| Classification System | Classification Symbols |
| IPC | A61K37/24 |

Documentation Searched other than Minimum Documentation
to the Extent that such Documents are Included in the Fields Searched [8]

**III. DOCUMENTS CONSIDERED TO BE RELEVANT [9]**

| Category * | Citation of Document, [11] with indication, where appropriate, of the relevant passages [12] | Relevant to Claim No. [13] |
|---|---|---|
| P | EP, A, 369437 (Kyowa Hakko Kogyo Co., Ltd.), 23 May 1990 (23. 05. 90), Claim (Family: none) | 1 - 7 |
| A | JP, A, 55-145615 (Behringwerke AG), 13 November 1980 (13. 11. 80), Claim & EP, A, 18561 | 1 - 7 |

* Special categories of cited documents: [10]

"A" document defining the general state of the art which is not considered to be of particular relevance

"E" earlier document but published on or after the International filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| September 11, 1990 (11. 09. 90) | October 1, 1990 (01. 10. 90) |
| International Searching Authority | Signature of Authorized Officer |
| Japanese Patent Office | |

Form PCT/ISA/210 (second sheet) (January 1985)